# EUROPÄISCHE PATENTSCHRIFT

(11) **EP 0 744 180 B1**
(45) Veröffentlichungstag und Bekanntmachung des Hinweises auf die Patenterteilung: **02.01.2003**
(21) Anmeldenummer: 96106742.8
(22) Anmeldetag: 29.04.1996
(51) Int. Cl.: A61M 1/06

(54) **Muttermilchpumpe**
Breast pump
Tire-lait

(30) Priorität: 26.05.1995 CH 155795
(43) Veröffentlichungstag der Anmeldung: 27.11.1996
(73) Patentinhaber: TRIMED AG, 9495 Triesen (LI); Medela AG Medical Technology, 6340 Baar (CH)
(72) Erfinder: Niederberger, Anton, 6370 Oberdorf NW (CH)
(74) Vertreter: Révy von Belvárd, Peter

(56) Entgegenhaltungen:
- WO-A-96/25187
- US-A- 3 238 937
- US-A- 4 607 596
- US-A- 5 304 129

## Beschreibung

Die vorliegende Erfindung betrifft eine Muttermilchpumpe .

Bekannte Muttermilchpumpen dienen zum Absaugen und Aufbewahren der Muttermilch, so dass ein Säugling auch ernährt werden kann, wenn seine Mutter nicht anwesend ist. Sie werden aber auch verwendet, wenn der Säugling Schwierigkeiten beim Saugen an der Mutterbrust hat oder wenn die Mutter zuviel oder zuwenig Muttermilch produziert.

In den US-A-4,929,229 und -4,857,051 ist eine Muttermilchpumpe offenbart, die einen auf eine Brust aufsetzbaren trichterförmigen Brustkörper aufweist, der über eine Saugleitung mit einer Kolbenpumpe verbunden ist, wobei in der Saugleitung ein Milchauffangbehälter angeordnet ist. Beim Saughub erzeugt die von Hand oder mit einem Elektromotor antreibbare Kolbenpumpe das Vakuum.

Obwohl sich diese Muttermilchpumpe bewährt hat, weist sie auch Nachteile auf. So ist diese Muttermilchpumpe teuer in der Anschaffung, relativ schwer und umständlich in der bloss manuell möglichen Handhabung. Auch lässt sich, schon wegen der umständlichen Handhabung, jeweils nur eine Brust absaugen, was natürlich die benötigte Zeit entsprechend verlängert.

Zwar ist auch eine motorisch antreibbare Pumpe aus der US-A-4,964,851 bekannt, doch ist auch dort nur die Absaugung einer Brust vorgesehen. Selbst wenn man diese Pumpe umbauen wollte, wofür diese US-A- keinerlei Anhaltspunkt gibt, so würden sich die beiden Luftwege derart gegenseitig beeinflussen, dass ein gleichmässiger Unterdruck nicht erzeugt werden könnte, was wiederum die Gefahr von Verletzungen und Entzündungen der Brust mit sich bringt.

Der Erfindung liegt daher die Aufgabe zugrunde, eine preisgünstige Muttermilchpumpe zu schaffen, die leicht im Gewicht und einfach zu handhaben ist und mit der ein Absaugen der Milch an beiden Brüsten gleichzeitig möglich ist, wobei Verletzungen und Entzündungen der Mutterbrust vermieden werden, die sich aus zu heftigem Saugen ergeben können. Diese Aufgabe wird durch die Merkmale des Anspruches 1 gelöst. Somit wird der Zeitaufwand zum Absaugen der Muttermilch - im Gegensatze zum genannten Stand der Technik - verringert, wo die Pumpe nur an jeder Brust einzeln und nacheinander verwendet werden konnte. Anderseits werden durch den hermetischen Abschluss der beiden Anschlüsse vermieden, dass bei Verwendung nur eines Brustkörpers bzw. während des Saugzyklus eine gegenseitige Beeinflussung des Saugdruckes in den beiden Anschlüssen erfolgt, was - bei dabei entstehendem zu hohen Saugdruck - zu den erwähnten Verletzungen führen kann.

Gemäss Anspruch 2 wird durch den alternierenden Anschluss erreicht, dass die Pumpe nicht gleichzeitig die gesamte Saugkraft aufbringen muss, sondern diese Kraft zeitlich aufgeteilt wird. Dies trägt dazu bei, dass jeder Brustkörper gleichmässig und jeweils unabhängig vom anderen betrieben werden kann.

Eine Anpassung an den natürlichen Saugzyklus eines Säuglings erfolgt zweckmässig bei einer Ausbildung nach Anspruch 4, wobei eine individuelle Anpassung durch die Ausgestaltung nach Anspruch 5 erreicht werden kann.

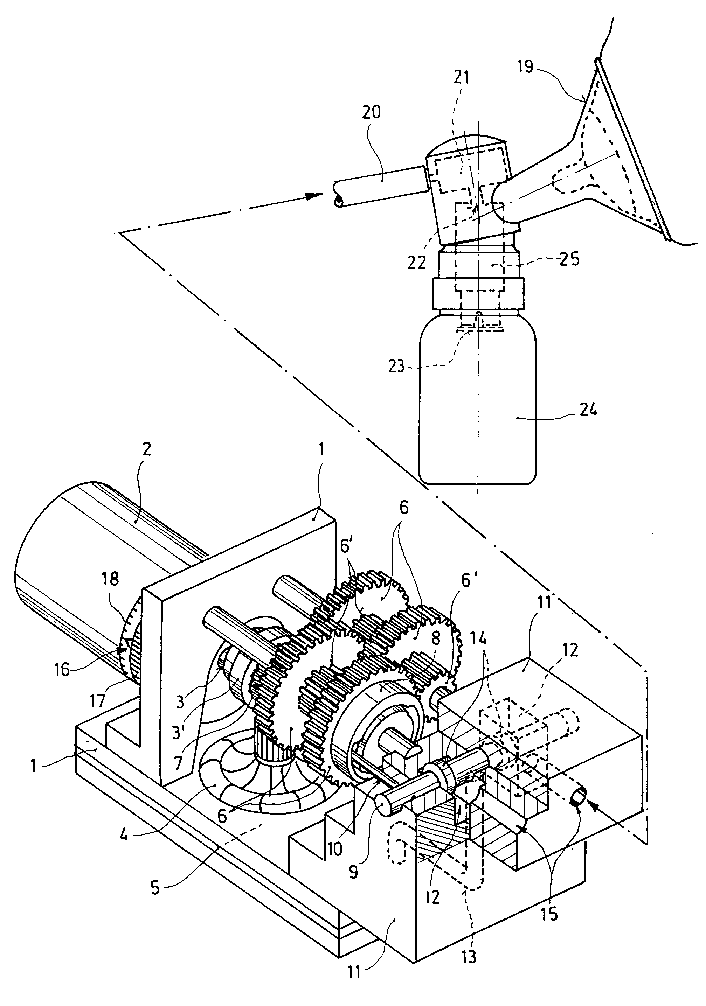

Nachfolgend wird ein Ausführungsbeispiel der erfindungsgemässen Muttermilchpumpe an Hand der einzigen Zeichnungsfigur näher erläutert. Diese zeigt rein schematisch eine teilweise geschnittene perspektivische Ansicht der Muttermilchpumpe.

Ein mit einem Komponententräger 1 der Muttermilchpumpe verbundener Elektromotor 2 treibt eine Antriebswelle 3, deren Ende als Exzenterantrieb 3' zum Antrieb einer bekannten Membranpumpe 4 ausgebildet ist. Die Membranpumpe 4 evakuiert einen im Boden des Trägers 1 angeordneten Vakuumraum 5. Es versteht sich, dass an Stelle einer Membranpumpe 4 auch eine Kolbenpumpe oder eine andere Pumpe, wie eine peristaltische Pumpe, verwendet werden könnte, doch haben Versuche gezeigt, dass eine Membranpumpe für den gedachten Zweck günstiger ist. Der Vakuumraum 5 "integriert" die Pumpimpulse der Membranpumpe und trägt damit zur Sicherstellung eines gleichmässigen Unterdruckes bzw. zur störungsfreien Umwandlung in die durch die später zu beschreibende Steuereinrichtung bestimmten gleichmässigen Saugzyklen bei.

Im Komponententräger 1 sind auf zwei zueinander parallele Wellen Zahnräder 6, 6' eines Untersetzungsgetriebes gelagert, die von einem ebenfalls auf der Antriebswelle 3 gelagerten Zahnrad 7 dieses Getriebes angetrieben werden. Das Untersetzungsgetriebe 6, 6' könnte auch als Riemengetriebe, beispielsweise mit Keilriemen, mindestens zum Teil, ausgebildet sein, um eine Einstellung der Zykluszeit über einen entsprechenden Einstellmechanismus, beispielsweise nach Art eines PIV-Getriebes, zu ermöglichen. Eine andere Art der Drehzahleinstellung kann darin bestehen, dass im Stromkreis des Motors 2 ein Potentiometer 16 angeordnet ist, das mittels eines Handrades 17 entlang einer Skala 18 zur Einstellung der Drehzahl des Motors 2 verstellbar ist, um die Saugcharakteristik und die Zykluszeit entsprechend anpassen zu können.

Am Ende des Untersetzungsgetriebes 6, 6' ist eine Steuereinrichtung 8-10 vorgesehen, die an sich auch bei Handbetätigung an Stelle der motorisch angetriebenen Pumpe vorgesehen sein könnte und dann beispielsweise als pneumatische Steuereinrichtung mit durch ein Drosselventil bestimmten Zykluseinstellung (z.B. zur Einstellung der Rücklaufzeit des Pumpkolbens) ausgebildet wäre.

Vorzugsweise besitzt die Steuereinrichtung 8-10 jedoch eine nockenartige Steuerscheibe 8, in deren Steuernut eine mit einer Steuerwelle 9 verbundene Steuerstange 10 eingreift, wobei die Steuerwelle 9 als steuerbares Ventilglied annähernd senkrecht zur Steuerstange 10 von dieser alternierend hin und her bewegt wird. Ein Teil des Komponententrägers 1 ist als Steuerblock 11 ausgebildet, der zwei identische Vakuumkammern 12 aufweist, die durch die alternierende Translationsbewegung der Steuerwelle 9 geöffnet und geschlossen werden und im offenen Zustand über eine in der Mitte zwischen ihnen mündende Vakuumleitung 13 oder über je eine Vakuumleitung 13 mit dem Vakuumraum 5 verbunden sind. Theoretisch wäre es möglich, das Ventilglied 9 unmittelbar im Vakuumraum 5 unterzubringen, so dass eine gesonderte Vakuumkammer 12 entfällt , was jedoch kein Ausführungsbeispiel der Erfindung ist. Eine weitere Alternative könnte darin bestehen, dass das Ventilglied 9 statt einer translatorischen Bewegung eine Drehung um seine eigene Achse, z.B. mittels eines an ihm befestigten Ritzels und einer hin- und hergehenden Zahnstange, ausführt, wobei die später beschriebenen Steuervorsprünge 14, statt flanschartig, sektorförmig sind, um den Kanal 13 und/oder 15 in der einen Drehstellung freizugeben, in der anderen zu verschliessen.

Um die beiden Vakuumkammern 12 in allen Betriebslagen hermetisch voneinander und vom Leitungssystem 13, 15 abzutrennen und so eine gegenseitige Beeinflussung der beiden Luftwege zu vermeiden, sind auf der Steuerwelle 9 in entsprechendem Abstand zwei Dichtelemente oder Steuervorsprünge 14 angeordnet. Jede Vakuumkammer 12 ist mit einer Auslassbohrung 15 versehen, in die je ein am Ende mit einem trichterförmigen Brustkörper 19 verbundener Saugschlauch 20 mündet. Alternativ wirken die beiden Steuervorsprünge 14 mit der jeweiligen Auslassbohrung 15 des Leitungssystems zusammen. Das mit dem Brustkörper 19 verbundene Ende des Saugschlauches 20 mündet in eine Saugkammer 21, die über eine Öffnung 22 mit einem durch ein Rückschlagventil 23 gegen einen Milchsammelbehälter 24 hin abgeschlossenen Saugraum 25 in Verbindung steht.

Obwohl nur ein einziger Brustkörper 19 gezeigt ist, versteht es sich, dass bei der Ausführungsform der Erfindung jede der gezeigten Auslassbohrungen 15 mit je einem Brustkörper 19 verbunden ist, wobei auch entweder je ein Milchsammelbehälter 24 oder ein gemeinsamer Milchsammelbehälter vorgesehen ist. In letzterem Falle könnten die beiden Brustkörper 19 miteinander gegebenenfalls verbunden sein, gewünschtenfalls in einem einstellbaren Abstand, z.B. nach Art eines Büstenhalters. Es ist ersichtlich, dass die Steuerstange 9 ein gemeinsames Ventil- bzw. Betätigungsglied für beide Leitungssysteme 13, 15 darstellt. Alternativ könnten gesonderte Ventilglieder für jedes der Leitungsysteme 13, 15 vorgesehen werden, doch ist die gemeinsame Betätigung über die Steuerstange 9 einfacher und unproblematischer. Auch könnten andere Arten von Ventilen, wie Ventile mit piezoelektrischen Verschlussgliedern, verwendet werden. Ferner kann der Vakuumraum 5 mit einem verstellbaren Drosselventil zur Regelung der Höhe des Saugdruckes versehen werden. Das Drosselventil führt dabei entweder nach aussen und dient als einstellbares Überdruckventil, falls der Unterdruck im Vakuumraum 5 ein eingestellte Niveau überschreitet, bevorzugt wird es jedoch in das Leitungssystem 12, 13, 15 eingebaut, um den diesem System vom Vakuumraum 5 her zugeführten Druck einzustellen.

Es versteht sich, dass die Steuereinrichtung im Rahmen der Erfindung beispielsweise auch als elektronische Steuereinrichtung ausgebildet werden kann, wobei beispielsweise das verstärkte Ausgangssignal einer astabilen Kippschaltung mit einem dem Steuerglied 9 entsprechenden Ventilglied verbunden ist. Theoretisch wäre es auch denkbar, die Drehzahl des Elektromotors 2 einstellbar zu machen, wie dies oben an Hand des Drehpotentiometers 16 beschrieben wurde. Bei der Benutzung eines einzigen Brustkörpers 19 könnte die Steuereinrichtung sogar einen Schalter im Stromkreis des Motors 2 umfassen, um diesen zyklisch ein- und auszuschalten und so eine Saugfrequenz von maximal 60 mal pro Minute zu erhalten. Die dargestellte Steuereinrichtung 8-10 zeichnet sich jedenfalls durch besondere Robustheit aus.

Der Elektromotor 2 wird aus Sicherheitsgründen bevorzugt mit maximal 12 Volt, z.B. auch nur mit Batteriestrom von 3 V, betrieben und ist zweckmässig über einen Transformator ans Netz anschliessbar, es wäre aber auch der erwähnte Batterieantrieb denkbar. Der Elektromotor 2 dreht sich in der Minute mit 3000 bis 6000, z.B. mit 5000, Umdrehungen, so dass auch die Membran der Membranpumpe 4 über den Exzenter 3' in der Minute entsprechend oft gehoben und gesenkt wird. Die Zahnräder 6, 6' des Untersetzungsgetriebes sind so aufeinander abgestimmt, dass die Steuerscheibe 8 die Steuerwelle 9 über die Steuerstange 10 im gewünschten Saugrhythmus von maximal 60, beispielsweise 40, Hin- und Herbewegungen pro Minute verschiebt. Gleichzeitig wird von der Steuerscheibe 8 die Zykluszeit der Steuerstange 9 gesteuert, während welcher die eine Vakuumkammer 12 geöffnet und die andere geschlossen ist. Dabei ist die Steuerscheibe 8 vorzugsweise so ausgebildet, dass die von der Steuereinrichtung 8-10 bestimmte Zykluszeit für beide Brustkörper 19 gleich ist (d.h. die Zeit des Öffnens und Schliessens jeder Vakuumkammer 12 ist gleich lang), obwohl auch eine Betriebsweise mit ungleichen Zyklen denkbar wäre. Für die letztere Betriebsart könnte die Steuerscheibe 8 beispielsweise lösbar und austauschbar mit dem ihr benachbarten Zahnrad 6 verbunden, beispielsweise formschlüssig, wie mit Stiften, aufgesteckt sein, um auch dadurch die Zykluszeit bzw. das Verhältnis der Zykluszeiten einstellen zu können.

Auf diese Weise wird jede Vakuumkammer 12 pro Minute höchstens 60 mal, z.B. 40 mal, mit dem Vakuumraum 5 verbunden, so dass jeder mit der Mutterbrust verbundene Brustkörper 19 auch maximal 60 mal, z.B. 40 mal, pro Minute durch Vakuumbeaufschlagung die Muttermilch absaugt, die auf bekannte Weise durch Schwerkraft in den mit dem Brustkörper 19 oder dem Saugschlauch 20 verbundenen Milchauffangbehälter 24 geleitet wird. Um eine besonders leichte Muttermilchpumpe zu erhalten, sind so viele Teile wie möglich aus Kunststoff hergestellt.

Falls aus irgendeinem Grunde doch nur aus einer Mutterbrust Milch abgesaugt werden sollte, ist dies durch Aufsetzen nur eines Brustkörpers 19 ohne weiteres möglich.

Es ist ersichtlich, dass die Steuerscheibe 8 zweckmässig so ausgebildet wird, dass ein plötzliches Umschalten des Ventilgliedes 9 erfolgt, so dass das Ventil 9, 10 als Schaltventil wirkt, das den vom Vakuumraum 5 bereitgestellten, gegebenenfalls gedrosselten, "integrierten" Druck damit unbeeinflusst von der jeweils anderen Vakuumkammer 12 weitergibt. Aus diesem Grund ist auch das gemeinsame Ventilglied 9 von besonderer erfinderischer Bedeutung, weil sich so Synchronisationsprobleme, welche bei gesonderten Ventilen auftreten würden, vermeiden lassen.

## Patentansprüche

1. Muttermilchpumpe, welche folgendes umfaßt:
a) zwei etwa trichterförmige Brustkörper (19);
b) die über je eine Saugleitung (20) an je eine Vakuumkammer (12) in einem ein Ventilglied aufnehmenden Steuerblock (11) angeschlossen sind, der die beiden Anschlüsse (15) für die Verbindung der jeweiligen Saugleitung (20) mit je einem Brustkörper (19) aufweist;
c) einen von der Saugleitung (20) über ein Rückschlagventil (23) getrennten Milchauffangbehälter (24);
d) einen Vakuumraum (5), der einerseits mit einer Pumpe und anderseits mit den beiden Vakuumkammern (12) in Verbindung steht, und in welchem ein vorbestimmter Vakuumdruck aufgebaut wird, um Muttermilch über das Rückschlagventil (23) in den Milchauffangbehälter (24) zu pumpen, wobei die beiden in die Vakuumkammern (12) mündenden Anschlüsse (15) durch ein steuerbares Ventilglied (9, 14) voneinander hermetisch trennbar sind.

2. Muttermilchpumpe nach Anspruch 1, **dadurch gekennzeichnet, dass** eine Steuereinrichtung (8-10) vorgesehen ist, durch die jeder Anschluss (15) von der Vakuumquelle (5) alternierend mit Vakuum beaufschlagt wird.

3. Muttermilchpumpe nach Anspruch 2, **dadurch gekennzeichnet, dass** ein Elektromotor (2) für den Antrieb der Steuereinrichtung (8-10) und/oder der Pumpe vorgesehen ist, und dass der Motor (2) vorzugsweise über eine Antriebswelle (3) und einen Exzenterantrieb (3') eine Membranpumpe (4) betätigt, die den Vakuumraum (5) als Vakuumquelle evakuiert, der über eine Vakuumleitung (13) mit Hilfe der Steuereinrichtung (8-10) alternierend mit je einer der Vakuumkammern (12) verbindbar ist,
und/oder
die Steuereinrichtung (8-10) mittels eines Motors (2) angetrieben ist, so dass der Brustkörper (19) zyklisch, mit Unterbrechungen von der Vakuumquelle (5) mit Vakuum beaufschlagt wird.

4. Muttermilchpumpe nach einem der Ansprüche 2 bis 3, **dadurch gekennzeichnet, dass** über die Steuereinrichtung (8-10) eine Zykluszeit von maximal 60 Saugzyklen pro Minute, insbesondere 30 bis 50 mal pro Minute, z.B. 40 mal pro Minute, steuerbar ist.

5. Muttermilchpumpe nach einem der Ansprüche 2 bis 4, **dadurch gekennzeichnet, dass** der Steuereinrichtung (8-10) eine Einstelleinrichtung (16-18) zur Einstellung der Zykluszeit zugeordnet ist.

6. Muttermilchpumpe nach einem der Ansprüche 2 bis 5, **dadurch gekennzeichnet, dass** die von der Steuereinrichtung (8-10) bestimmte Zykluszeit für beide Brustkörper (19) gleich ist, und dass vorzugsweise zum zyklischen Beaufschlagen mit Vakuum eine Ventilanordnung (12-15) von der Steuereinrichtung (8-10) steuerbar ist, bevorzugt pro Anschluss (15, 20) für einen Brustkörper (19) ein gemeinsames Betätigungsorgan (9) vorgesehen ist, wobei zweckmässig zum hermetischen Trennen der beiden Vakuumkammern (12) voneinander auf der Steuerwelle (9) im entsprechenden Abstand zwei Dichtelemente (14) angeordnet sind.

7. Muttermilchpumpe nach einem der Ansprüche 2 bis 6, **dadurch gekennzeichnet, dass** die Steuereinrichtung (8-10) eine von einer Antriebswelle (3), und gegebenenfalls einem Untersetzungsgetriebe (6, 6'), betriebenes Ventilglied (9) aufweist, und dass vorzugsweise die Zykluszeit durch eine Steuerscheibe (8) der Steuereinrichtung (8-10) steuerbar ist

8. Muttermilchpumpe nach Anspruch 7, **dadurch gekennzeichnet, dass** das Ventilglied (9) als über ein in eine Nut der Steuerscheibe (8) eingreifende Steuerstange (10) hin- und herbewegbares Steuerglied (9) zum alternierenden Verbinden der Vakuumkammern (12) mit der Vakuumleitung (13) ausgebildet ist,
und dass vorzugsweise die Antriebswelle (3) mit 3000 bis 6000, insbesondere 5000, Umdrehungen pro Minute angetrieben wird.

9. Muttermilchpumpe nach einem der vorhergehenden Ansprüche, **dadurch gekennzeichnet, dass** sie als Membranpumpe (4) ausgebildet ist.

## Claims

1. Breast pump, comprising
a) two funnelshaped breast elements (19),
b) which are each connected via a vacuum pipe (20) to a respective vacuum chamber (12) located in a control block (11) which accommodates a valve member and includes the two connections (15) for joining the respective vacuum pipe (20) to a respective breast element,
c) a milk collecting container (24) which is separated from the suction pipe (20) via a non-return valve (23),
d) a vacuum chamber (5) which is on the one side connected to a pump and on the other side to the two vacuum chambers (12), and wherein a predetermined vacuum pressure is built up for pumping breast milk via the non-return valve (23) into the milk collecting container (24), and the two connections (15) which terminate in the vacuum chambers are hermetically separable from each other by a controllable valve member (9, 14).

2. Breast pump according to Claim 1, **characterised in that** a control device (8-10) is provided by means of which each connection (15) is alternately vacuum loaded by the vacuum source (5).

3. Breast pump according to Claim 2, **characterised in that** an electric motor (2) is provided for driving the control device (8-10) and/or the pump, and the motor (2) operates preferably via a driveshaft (3) and an eccentric drive (3') a diaphragm pump (4) which evacuates the vacuum chamber (5) as vacuum source which is alternately connectable to a respective vacuum chamber (12) via a vacuum pipe (13) and with the aid of the control device (8-10),
and/or
the control device (8-10) is driven by means of a motor (2) so that the breast elements (19) are cyclically loaded with a vacuum with interruptions by the vacuum source (5).

4. Breast pump according to one of Claims 2 to 3, **characterised in that** a cycle period of maximum 60 suction cycles per minute, in particular between 30 and 50 times per minute, for example 40 times per minute, is controlled via the control device (8-10).

5. Breast pump according to one of Claims 2 to 4, **characterised in that** the control device (8-10) is associated with a setting device (16-18) for setting the cycle period.

6. Breast pump according to one of Claims 2 to 5, **characterised in that** the cycle period determined by the control device (8-10) is identical for both breast elements (19), and preferably a valve arrangement (12-15) is controllable by the control device (8-10) for cyclic loading with vacuum, and preferably a joint operating element (9) is provided per connection (15, 20) for a breast element (19), and for practical reasons two sealing elements (14) are placed at a respective distance from each other on the control shaft (9) for the purpose of hermetic separation of both vacuum chambers (12).

7. Breast pump according to one of Claims 2 to 6, **characterised in that** the control device (8-10) comprises a valve element (9) which is operated by a driveshaft (3) and, if appropriate, a reduction gearing (6, 6'), and the cycle period is preferably controllable by a control disc (8) of the control device (8-10).

8. Breast pump according to Claim 7, **characterised in that** the valve member (9) is designed as a control element (9) which is movable to and fro via a control bar (10) which engages a groove of the control disc (8) for the purpose of alternating connection of the vacuum chambers (12) to the vacuum pipe (13), and the driveshaft (3) is preferably driven with 3,000 to 6,000, in particular 5,000, revolutions per minute.

9. Breast pump according to one of the above claims, **characterised in that** it is designed as a diaphragm pump (4).

## Revendications

1. Pompe pour tire-lait maternel ou tire-lait, comprenant les éléments suivant :
a) deux corps pour sein (19) à peu près en forme d'entonnoir ;
b) raccordés, chacun par une conduite d'aspiration (20), chaque fois à une chambre à vide (12), dans un bloc de commande (11) logeant un organe de soupape, bloc de commande présentant les deux raccordements (15) pour assurer la liaison de la conduite d'aspiration (20) respective, chacun à un corps pour sein (19) ;
c) un récipient de captage de lait (24) séparé de la conduite d'aspiration (20) par l'intermédiaire d'un clapet anti-retour (23) ;
d) une enceinte à vide (5) reliée, d'une part, à une pompe et, d'autre part, aux deux chambres à vide (12) et dans laquelle est établi un vide de niveau prédéterminé pour pomper le lait maternel, en passant par le clapet anti-retour (23), dans le récipient de captage de lait (24), les deux raccordements (15) débouchant dans la chambre à vide (12) étant séparable hermétiquement l'un de l'autre au moyen d'un organe de soupape (9, 14) pouvant être commandé.

2. Pompe pour tire-lait selon la revendication 1, **caractérisée en ce que** un dispositif de commande (8-10) est prévu, au moyen duquel chaque raccordement (15) est sollicité en alternance par un vide, depuis la source de vide (5).

3. Pompe pour tire-lait selon la revendication 2, **caractérisée en ce qu'**un moteur électrique (2) devant assurer l'entraînement du dispositif de commande (8-10) et/ou de la pompe est prévu, et **en ce que** le moteur (2) actionne une pompe à membrane (4) de préférence par l'intermédiaire d'un arbre d'entraînement (3) et d'un entraînement à excentrique (3'), la pompe à membrane mettant sous vide, à titre de source de vide, l'enceinte à vide (5) qui est susceptible d'être reliée de façon alternée à chacune des chambre à vide (12) à l'aide d'un dispositif de commande (8-10), et/ou
le dispositif de commande (8-10) est entraîné à l'aide d'un moteur (2) de manière que le corps pour sein (19) soit sollicité cycliquement par un vide, avec des interruptions vis à vis de la source de vide (5).

4. Pompe pour tire-lait selon l'une des revendications 2 à 3, **caractérisée en ce que**, par l'intermédiaire du dispositif de commande (8-10), on peut commander un temps de cycle maximal de 60 cycles par minutes, en particulier de 30 à 50 fois par minute, par exemple 40 fois par minute.

5. Pompe pour tire-lait selon l'une des revendications 2 à 4, **caractérisé en ce qu'**au dispositif de commande (8-10) est associé un dispositif de réglage (16-18) pour régler le temps de cycle.

6. Pompe pour tire-lait selon l'une des revendications 2 à 5, **caractérisée en ce que** le temps de cycle, déterminé par le dispositif de commande (8-10) pour les deux corps pour sein (19), est identique et **en ce que**, de préférence, pour obtenir une sollicitation cyclique par le vide, un agencement à soupape (12-15) peut être commandé par le dispositif de commande (8-10), de préférence par raccordement (15, 20) pour un corps pour sein (19) est prévu un organe d'actionnement (9) commun, sachant que, de manière appropriée, pour obtenir une séparation hermétique des deux chambres à vide (12) l'une de l'autre, sur l'arbre de commande (9) sont disposés deux éléments d'étanchéité (14) placés à distance correspondante.

7. Pompe pour tire-lait selon l'une des revendications 2 à 6, **caractérisée en ce que** le dispositif de commande (8-10) présente un organe de soupape (9) actionné par un arbre d'entraînement (3) et, le cas échéant, une transmission de démultiplication (6, 6'), et **en ce que**, de préférence, le temps de cycle peut être commandé par un disque de commande (8) du dispositif de commande (8 à 10).

8. Pompe pour tire-lait selon la revendication 7, **caractérisée en ce que** l'organe de soupape (9) est réalisé sous la forme d'un organe de commande (9), pouvant être déplacé dans un sens ou dans l'autre, par l'intermédiaire d'une tige de commande (10) s'engageant dans une rainure du disque de commande (8), pour obtenir une liaison alternée des chambres à vide (12) à la conduite à vide (13),
et **en ce que**, de préférence, l'arbre d'entraînement (3) est entraîné à une vitesse de rotation de 3000 à 6000, en particulier de 5000 tours par minute.

9. Pompe pour tire-lait selon l'une des revendications précédentes, **caractérisée en ce qu'**elle est réalisée sous la forme de pompe à membrane (4).
